# EUROPEAN PATENT APPLICATION

(11) **EP 1 634 588 A1**
(43) Date of publication of application: **15.03.2006**
(21) Application number: 03817077.5
(22) Date of filing: 14.08.2003
(51) Int. Cl.: A61K 31/045, A61K 31/11, A61P 19/10, A61P 43/00, A23L 1/30

(54) **COMPOSITIONS AND FOODS AND DRINKS CONTIAING HIGHER FATTY ACID DERIVATIVE**

(30) Priority: 28.05.2003 JP 2003151066
(71) Applicant: Eisai Co., Ltd., Tokyo 112-8088 (JP)
(72) Inventor: CHINEN, Isao, Naha-shi, Okinawa 903-0821 (JP)
(74) Representative: Daniels, Jeffrey Nicholas
(86) International application number: PCT/JP2003/010347
(87) International publication number: WO 2004/105739

(57) **Abstract**

An object of the invention is to provide a composition containing a higher fatty acid derivative, which can be easily produced at low cost, is effective as an agent for prevention and treatment of osteoporosis induced by various factors such as diet, dietary restriction, etc., is highly safe, and can be used regularly; and foods and drinks comprising the composition, which is effective as an agent for prevention and treatment of osteoporosis induced by various factors such as diet, dietary restriction, etc., and suitable as foods and drinks for hospitals, healthy drinks or foods, feeds, etc.

The composition containing a higher fatty acid derivative comprises one kind of higher fatty acid derivatives as an active ingredient and is used as an agent for prevention and treatment of osteoporosis. The higher fatty acid derivatives include octacosanal. The foods and drinks comprise the composition with a higher fatty acid derivative.

## Description

### Technical Field

The present invention relates to a composition containing a higher fatty acid derivative as an agent for prevention and treatment of osteoporosis, which can be easily produced at low cost, is highly safe and can be used regularly; and it relates to foods and drinks which comprise the composition and can be used regularly as foods and drinks for hospitals, healthy drinks or foods, safe feeds, etc.

### Background Art

Recently, the number of the patients suffering from osteoporosis has increased due to various factors such as diet, change in what people eat, and longer life spans, which has become a problem. This osteoporosis develops due to a reduced secretion of hormones following menopause, a disorder of inappropriate secretion of hormone, etc., and is a disorder in which bone density is reduced, resulting in easy fracture of bones. In pazticular, older patients who have broken their femur recover slowly, and bone fracture is placed as the third cause, behind stroke and senile weakness, by which elderly people become bedridden patients. Thus, osteoporosis is a big social problem.

The most serious problems to osteoporosis patients are easy bone fracture and pain, and the patients diagnosed as osteoporosis by a doctor undergo hormone replacement therapy in which estrogen is administered, or are administered drugs such as bisphosphonate, raloxifene, calcitonin, calcium tablet, vitamin D, vitamin K, and ipriflavone.

In case of potential patients who have a slight degree of reduced bone density, however, clear symptoms do not appear. Thus, there are no motives for the potential patients to see a doctor, and in many cases, potential patients are not recognized. In Japan, it is said that there are about as many as five million potential patients. According to one report, especially of women, about 20% in their fifties have reduced bone density, about 48% in their sixties, about 70% in their seventies, and about 85% in their eighties.

Under the present situation in which there exists a large number of potential osteoporosis patients, it can be said that it is preferable to ingest foods, agents, or the like which are effective for the prevention and treatment of osteoporosis but have milder effect than medicines prescribed by a doctor, and which are easily taken through a daily diet, healthy foods, supplements, etc. In view of such present situation, various kinds of agents for prevention and treatment of osteoporosis and healthy foods have been proposed. For example, it has been proposed that octacosanol-containing white sugarcane native to Brazil has female hormone effect (Japanese Patent Application Laid-Open (JP-A) No. 63-283552). Further, an agent for prevention and treatment of osteoporosis, whose active ingredient is one kind or two or more kinds of linear saturated alcohols having a carbon number of 22 to 38, has been proposed (Japanese Patent Application Laid-Open (JP-A) No. 04-266820).

On the other hand, other proposals, focusing on another effect of the octacosanol, have been made. It has been proposed that the octacosanol has an effect to improve one's strength and to stimulate sex hormone (Japanese Patent Application Laid-Open (JP-A) No. 04-278061), and to reduce cholesterol level (Japanese Patent Application Laid-Open (JP-A) No. 07-505414). Further, Japanese Patent Application Laid-Open (JP-A) No. 01-225461 focuses on the effect achieved by octacosanal similar to the octacosanol and proposes a nutritional food having an effect on improvement in physical strength and on recovery from fatigue. Further, it has been proposed that linear aliphatic aldehyde having a carbon number of 27 or the like has an effect to lower the cholesterol level (Japanese Patent Application Laid-Open (JP-A) No. 59-53427).

Various kinds of agents and the like for treatment of osteoporosis have been proposed; however, there has been further demands for compositions or foods and drinks which are novel, can be easily produced at low cost, are effective as an agent for prevention and treatment of osteoporosis induced by various factors such as diet, dietary restriction, etc., can be suitably used for various applications, are highly safe, and can be used regularly.

The challenge of the present invention is to meet the demand and to attain the following object That is, an object of the present invention is to provide a composition containing a higher fatty acid derivative which can be easily produced at low cost, is effective as an agent for prevention and treatment of osteoporosis induced by various factors such as diet, dietary restriction, etc., is highly safe, and can be used regularly; and foods and drinks which comprise the composition, are effective as an agent for prevention and treatment of osteoporosis induced by various factors such as diet, dietary restriction, etc., and are suitable as foods and drinks for hospitals, healthy drinks or foods, feeds, etc.

### Disclosure of Invention

The composition containing a higher fatty acid derivative of the invention includes at least one kind of higher fatty acid derivatives as an active ingredient and is used as an agent for prevention and treatment of osteoporosis.

The composition containing a higher fatty acid derivative can be suitably used as an agent for prevention and treatment of osteoporosis because the composition includes at least one kind of higher fatty acid derivatives as an active ingredient.

The foods and drinks of the invention comprise the composition containing a higher fatty acid derivative of the invention.

The foods and drinks comprise a natural ingredient as an active ingredient, and the foods and drinks are safe and are particularly suitable for foods and drinks for hospitals, daily use, feeds, etc.

### Best Mode for Carrying Out the Invention

### (Composition containing higher fatty acid derivative)

The composition containing a higher fatty acid derivative of the invention comprises one kind of higher fatty acid derivatives as an active ingredient, and may further comprise other components appropriately selected according to the purpose.

### - Higher fatty acid derivative -

The higher fatty acid derivative is not particularly limited as long as it is a derivative of higher fatty acid and can be appropriately selected according to the purpose. Examples thereof include higher fatty acids containing hydroxyl group, higher fatty acids containing aldehyde group, and the like.

The higher fatty acid containing hydroxyl group is not particularly limited as long as it has a hydroxyl group. Examples thereof include tetracosanol, hexacosanol, heptacvsanvl, octacosanol, nonacosanol, and the like. Among these, the octacosanol is preferred.

The aldehyde-containing higher fatty acid is not particularly limited as long as it has an aldehyde group. Examples thereof include tetracosanal, hexacosanal, heptacosanal, octacosanal, nonacosanal, and the like. Among these, the octacosanal is particularly preferred.

These may be used singly or two or more may be used in combination. Among these, the higher fatty acid containing aldehyde group is particularly preferred. When two or more of the higher fatty acid derivatives are used in combination, a combination (mixture) of the octacasanol and the octacosanal is preferred.

The higher fatty acid derivative may be a chemically synthesized compound or a naturally-occurring compound. The latter is advantageous for application to foods and drinks in terms of safety, and among them, natural product extracts are preferred.

The carbon number of the higher fatty acid derivative is not particularly limited and can be appropriately selected according to the purpose. For example, the carbon number is preferably 24 to 38, and more preferably 26 to 32.

The method for producing the higher fatty add derivative is not particularly limited and can be appropriately selected according to the purpose. For example, the higher fatty acid derivative is produced by extracting from natural products by a known synthetic method, or the like. Among these, the method of extraction from natural products is preferable since more convenient, low-cost production is possible.

The natural product is not particularly limited. Examples thereof include sugarcane, brown rice germ, malt, apple peel, palm fruit, and the like.

An example of the method for producing the octacosanal as the higher fatty acid derivative will be described below. That is, initially, using sugarcane as the natural product, the sugarcane skin is chopped into small pieces. Then, an organic solvent such as benzene, isopropanol and hexane is added; the mixture is heated for about 10 minutes to about several hours under the temperature condition of 30°C to 80°C and filtered. A fat-like substance, which is obtained by subjecting this filtrate to vacuum concentration, is loaded onto a silica gel column. The initial fraction, in which the fat-like substance is eluted in an organic solvent such as benzene, is further purified with a thin-layer chromatography, and fractions with Rf value of about 0.60 to 0.65 are collected and reaystallized. In this way, the octacosanal can be produced.

Alternatively, since the fat-like substance normally contains 60% by mass to 90% by mass of the octacosanal, the other components may be added to the fat-like substance, thereby enabling to formulate a drug without further treatment.

The content of the higher fatty acid derivative in the composition containing a higher fatty acid derivative is not particularly limited and can be appropriately selected according to the purpose.

### - Other components -

The other components are not particularly limited as long as they do not prevent the object of the invention, and they can be appropriately selected according to the purpose. Examples thereof include medicinal components, additives, and the like.

Examples of the medicinal component include herbal medicines, vitamins, antidiarrheic agents, medicine for intestinal disorders, antiulcer agents, stomachics and digestives, antacids, digestive enzymes, cough suppressants, cholagogues, antihistamines, antianxiety agents, antiepileptic agents, anti-pyretic, analgesic, and anti-inflammatory agents, muscle relaxants, diuretics, cardiotonic agents, hypotensive agents, vasodilating agents, antiarrhythmic agents, thrombolytic agents, antihyperlipidemic agents, bronchodilators, antasthmatics, antiallergic agents, antibiotics, antiviral agents, hormonal agents, and the like.

Examples of the herbal medicine include gambir, sweet hydrangea leaf, fennel, turmeric, Rabdosia japonica, scutellaria root, coptis rhiaome, Artemisiae folium, pueraria root, glycyrrhiza, cinnamon bark, geranium herb, Magnolia Bark, saffron, hawthorn, peony root, ginger, senna leaf, swertia herb, clove, citrus unshiu peel, bitter orange peel, atractyrodes rhizome, saposhnikovia root, ephedra herb, longgu, forsythia fruit, and the like. These may be added as an extract, or may be added as an extract or powder.

Examples of the vitamin compound include vitamin A group such as retinol and retinal; vitamin B group such as carotenes, thiamine, riboflavin, and biotin; vitamin C group such as ascorbin; vitamin D group such as cholecalciferol and ergocalciferol; vitamin E group such as α-tocopherol and tocotrienol; vitamin K group such as phytonadione and menatetrenone; nicotinic acid, pantothenic acid, folic acid, etc. and derivatives thereof or salts thereof, and the like.

The additive is not particularly limited. Examples thereof include stabilizing agents, surfactants, solubilizing agents, buffering agents, sweetening agents, flavoring agents, suspending agents, antioxidant agents, cooling agents, fragrant agents, emulsifying agents, pH adjusters, dispersants, aromatics, antiseptic agents, preservatives, and the like.

Examples of the stabilizing agent include adipic acid, ascorbic acid, sodium L-ascorbate, L-aspartic acid, sodium L-aspartate, sodium sulfite, L-arginine, sodium benzoate, inositol, disodium edetate, erythorbic acid, sodium erythorbate, sodium citrate, glycine, glycerin, glycerine fatty acid ester, light anhydrous silicic acid, tocopheryl acetate, gelatin, soybean oil unsaponifiables, polyoxyethylene polyoxypropylene glycol, anhydrous sodium citrate, glyceryl monostearate, sodium hydrogenphosphate, and the like.

Examples of the surfactant include cholesterol, sucrose fatty acid ester, stearyl alcohol, sorbitan sesquioleate, cetanol, sorbitan fatty acid ester, polyoxyethylene hydrogenated castor oil, polyoxyethylene polyoxypropylene glycol, polysorbate, macrogol, sorbitan monooleate, glyceryl monostearate, sorbitan monolaurate, sodium lauryl sulfate, lauromacrogol, and the like.

Examples of the solubilizing agent include L-aspartic acid, L-arginine, sodium benzoate, ethanol, sodium chloride sorbitan fatty acid ester, sodium hydrogen carbonate, polyoxyethylene hydrogenated castor oil, polyoxyethylene polyoxypropylene glycol, polysorbate, polyvinyl alcohol, D-mannitol, dehydrated ethanol, and the like.

Examples of the buffering agent include ascorbic acid, magnesium L-aspartate, aminoethylsulfonic acid, L-arginine, benzoic acid, sodium benzoate, citric acid, calcium citrate, sodium citrate, disodium citrate, glycine, sodium hydrogen carbonate, lactic acid, glucose, anhydrous citric acid, anhydrous sodium citrate, anhydrous dibasic sodium phosphate, sodium metaphosphate, sodium hydrogen phosphate, dipotassium hydrogen phosphate, potassium dihydrogen phosphate, sodium dihydrogen phosphate, and the like.

Examples of the sweetening agent include aspartame, sweet hydrangea leaf, glycynchiza, fructose, fructose-glucose solution, glucose-fructose syrup, reduced maltose syrup, dipotassium glycyrrhizinate, monoammonium glycyrrhizinate, acesulfame potassium, high fructose corn syrup, glucose, starch syrup, lactose, sucrose, purified sucrose, honey, saccharin, saccharin sodium, stevia, sucralvse, erythritol, xylitol, D-sorbitol, maltitol, maltose, D-mannitol, simple syrup, and the like.

Examples of the flavoring agent include ascorbic acid, L-aspartic acid, DL-alanine, disodium 5'-inosinate, fennel, sodium chloride, orange oil, cacao powder, camphor, glycine, glycerin, L-glutarnic acid, saffron, tartaric acid, ginger, D-sorbitol, mint, adipic acid, fumaric acid, peppermint, borneol menthol, borneol (ryuno), green tea powder, apple juice, apple-cider vinegar, lemon oil, rose oil, royal jelly, and the like.

Examples of the suspending agent include gum arabic, carrageenan, carboxy vinyl polymer, agar, xanthan gum, glycerine fatty acid ester, titanium oxide, sucrose fatty acid ester, stearyl alcohol, aluminium stearate, cetanol, purified gelatin, sorbitan fatty acid ester, soybean lecithin, propylene glycol fatty acid ester, pectin, propylene glycol, polyoxyethylene hydrogenated castor oil, polyoxyethylene hydrogenated castor oil, polyethyleneglycol, methylcellulose, glyceryl monostearate, liquid paraffin, and the like.

Examples of the antioxidant agent include ascorbic acid, L-ascorbyl stearate, sodium bisulfite, sodium sulfite, erythorbic acid, cysteine hydrochloride, tocopherol acetate, soybean lecithin, butylhydroxyanisol, propyl gallate, and the like.

Examples of the cooling agent include geraniol, mentha water, mentha oil, borneol, menthol, mint, and the like.

Examples of the fragrant agent include fennel, ethyl vanillin, ethyl maltol, orange, camphor, cinnamon, sugar flavor, cirtnamaldehyde, cherry flavor, orange peel tincture, mint, vanilla flavor, vanillin, bitter essence, fruit flavor, flavor GI, vermouth flavor, mix flavor, mint flavor, menthol, eucalyptus oil, borneol (ryuno), lemon powder, lemon oil, rosin, rose oil, and the like.

Examples of the emulsifying agent include carrageenan, carboxy vinyl polymer, carmellose sodium, guar gum, glycerine fatty acid ester, distearate polyethylene glycol, sucrose fatty acid ester, stearyl alcohol, stearic acid, cetanol, gelatin, sorbitan fatty acid ester, soybean lecithin, hydroxypropylcellulose, propylene glycol, propylene glycol fatty acid ester, polyoxyethylene polyoxypropylene glycol, polysorbate, myristyl alcohol glyceryl monostearate, sorbitan monolaurate, sodium lauryl sulfate, and the like.

Examples of the pH adjuster include dilute hydrochloric acid, sodium citrate, glycine, succinic acid, acetic acid, tartaric acid, sodium hydroxide, sodium hydrogen carbonate, sodium carbonate, monosodium fumarate, phosphoric acid, trisodium phosphate, sodium hydrogenphosphate, dipotassium hydrogen phosphate, sodium dihydrogen phosphate, and the like.

Examples of the dispersant include aminoalkyl methacrylate polymer RS, gum arabic, oleic acid, carboxy vinyl polymer, carmellose sodium, glycerin, crystalline cellulose, choline phosphate, lecithin, sorbitan sesquioleate, dextrin, corn starch, sorbitan trioleate, hydroxyethylcellulose, hydroxypropylcellulose, methylcellulose, glyceryl monostearate, sodium lauryl sulfate, liquid paraffin, and the like.

Examples of the aromatics include fennel, ethyl vanillin, orange oil, camphor, cinnamon" ginger, agalloch, clove, mint, vanilla, vanillin, borneol, maltol, menthol, menthol, eucalyptus, borneol (ryuno), lemon" rose water, and the like.

Examples of the antiseptic agent include aminoethylsulfonic acid, benzoic acid, sodium benzoate, ethanol, disodium edetate, agar, sodium citrate, sodium salicylate, sorbic acid, isopropyl p-hydroxybenzoate, ethyl p-hydroxybenzoate, menthol, and the like.

Examples of the preservative include sodium benzoate, disodium edetate, glycerin, salicylic acid, sodium salicylate, D-sorbitol, sorbic acid, isobutyl p-hydroxybenzoate, ethyl p-hydroxybenzoate, butyl p-hydroxybenzoate, methyl p-hydroxybenzoate, propylene glycol, and the like.

The form of the agent for prevention and treatment of osteoporosis is not particularly limited and can be appropriately selected according to the purpose. For example, the agent may have a solid formulation such as powder, fine granule, granule, and microcapsule. These may be further formed as a tablet, film-coated formulation, chewable tablet, and caplet, or may be filled in a capsule, etc. Alternatively, the agent for prevention and treatment of osteoporosis may have a liquid formulation, which is prepared by bringing the composition containing a higher fatty acid derivative into solution or suspension with an appropriately selected solvent,

The composition containing a higher fatty acid derivative of the invention can be conveniently taken on a daily basis, is highly safe at the same time, can effectively work on at least one of the prevention and treatment of osteoporosis, and can be particularly suitably used for the foods and drinks of the invention described below.

### (Foods and drinks)

The foods and drinks of the invention comprise the composition containing a higher fatty acid derivative, and further comprise other components appropriately selected according to necessity.

The application of the foods and drinks of the invention are not particularly limited and can be appropriately selected according to the purpose. The foods and drinks of the invention can be suitably used for various applications including, for example, foods, drinks, and feeds.

Suitable examples of the food include regular foods, hospital diets, functional foods for health such as dietary supplement and food for prevention and treatment of disease, and the like. Specific examples of these include frozen desserts such as ice cream, sherbet and shaved ice; noodles such as buckwheat noodle, wheat noodle, beanstarch vermicelli, pasta wrapping for Chinese meat dumpling, pasta wrapping for steamed dumpling, Chinese noodle, and instant noodle; confectioneries such sweet, candy, gum, chocolate, tablet candy (ramune), snack food, biscuit, jelly, jam, cream, baked cake, and bread; marine products such as crab, salmon, clam, tuna, sardine, shrimp, bonito, mackerel, whale, oyster, saury, squid, ark shell, scallop, ear shell, echinoid, salmon roe, ormer; processed marine or livestock foods such as steamed fish paste, ham, and sausage; dairy products such as processed milk and fermented milk; oils and fats, and processed foods of oils and fats, such as cooking oil, tempura oil, margarine, mayonnaise, shortening, whip cream, and dressing; seasonings such as sauce and baste; retort pouch foods such as curry, stew, oyakodon, rice porridge, rice and vegetable gruel, chukadon, katsudon, tempura bowl, eel bowl, rice with hashed meat, oden, mapo tofu, beef bow1, meat sauce, egg-drop soup, omuraisu, Chinese meat dumpling, steamed dumpling, hamburger, and meatball; and the like.

Suitable examples of the drink include regular drinks, hospital drinks, functional drinks for health such as nutritious supplement drink and drink for prevention and treatment of disease, and the like. Specific examples of these include soft drinks, carbonated drinks, fruit drinks, energy drinks, lactic acid drinks, and the like.

Suitable examples of the feed include feed for pigs, feed for cattle, feed for chickens, feed for horses, pet food, and the like. Examples of the pet food include pet food for dogs, pet food for cats, and the like.

The content of the composition containing a higher fatty acid derivative in the foods and drinks is not particularly limited and can be appropriately selected according to the purpose.

The foods and drinks of the invention can be conveniently taken on a daily basis, are highly safe at the same time, and are effective in at least one of the prevention and treatment of osteoporosis.

Hereinafter, examples of the invention will be described, however, it is to be understood that the invention is not limited to these examples.

### (Example 1)

### - Production of agent for prevention and treatment of osteoporosis -

The epidermis of a sugarcane as the natural product was peeled off with a peeler. To 30 g thereof, 600 ml of benzene was added, and the mixture was heated for 30 minutes under the temperature condition of 60°C with stirring. The heated solution of benzene was filtered, and the filtrate was subjected to vacuum concentration at 40°C under the flow of nitrogen gas until it was dried to obtain 7.2 g of white fat-like substance. Then, onto a column filled with silica gel (100 mesh or finer) which was had been washed with benzene beforehand, a solution in which the fat-like substance was dissolved in benzene was loaded and eluted with benzene. Each fraction was subjected to silica gel thin layer chromatography using a mixture of n-hexane and benzene [n-hexane : benzene = 1 : 1 (v/v)] as a developing solvent, and a fraction with Rf value of 0.62 was collected. The fraction was dried and concentrated under a nitrogen stream to obtain 2.1 g of crystallized wax (agent for prevention and treatment of osteoporosis A).

The crystallized wax was analyzed with gas chromatography, and it was found that it contained 23.9% by mass of octacosanal.

### (Example 2)

### - Production of agent for prevention and treatment of osteoporosis -

To 1.0 g of octacosanol (available from Tokyo Kasei Kogyo Co., Ltd.), 300 ml of CH₂Cl₂, 5.0 g of CaCO₃, and 2.0 g of molecular sieves (MS4A, available from Sanko Junyaku Co., Ltd.) were added, and the mixture was stirred for 30 minutes at room temperature. To this solution, 3.0 g of pyridinium chlorochromate was added and subjected to reaction for 2 hours at room temperature. Further, 5.0 g of CaCO₃, 10.0 g of diatom earth and 300 ml of hexane were added; the mixture was stirred for 15 minutes and then filtered to obtain roughly purified substance. The roughly purified substance was purified with an elution solvent of hexane and toluene (3 : 1, mass mixing ratio) using a silica gel column (2.0 x 60 cm) to obtain 456.8 mg of vctacosanal (agent for prevention and treatment of osteoporosis B) (yield: 45.9%).

The obtained octacosanal was subjected to ¹H-NMR (300 MHz) and mass spectrum analysis (EI method). The results are shown below.

### < ¹H-NMR chemical shift δ (assigned hydrogen) >

0.88 (3H, t, J 6.7 Hz), 1.63 (2H, m), 2.42 (2H., dt, J 2.0 and 7.0 Hz), 9.77 (1H, t, J 1.8 Hz)

### < Result of mass spectrum analysis >

Calculated value of molecular weight as C₂₈H₅₆O: 408.4331
Molecular weight peak of mass spectrum: 408.4326

### (Example 3 and Comparative Examples 1 and 2)

### - Production of feed -

A standard feed and restricted feed (1), which were composed of the compositions of the following Table 1, respectively, were produced by a common procedure. Further, a restricted feed (2) was produced by further adding the produced octacosanal (the above-mentioned agent for prevention and treatment of osteoporosis B) to the restricted feed (1) so that daily intake of octacosanal by rats was 7 mg per head, and each feed was evaluated as follows. The results are shown in Table 2.

**Table 1**

| | Comparative Example 1 | Comparative Example 2 | Example 3 Example 3 |
|---|---|---|---|
| | Standard Feed (g/100g) | Restricted Feed (1) (g/100g) | Restricted Feed (2) (g/100g) |
| Casein | 20.0 | 40.0 | 40.0 |
| β-cornstarch | 39.7 | 29.7 | 29.7 |
| a-cornstarch | 13.2 | 8.2 | 8.2 |
| Sucrose | 10.0 | 0.0 | 0.0 |
| Soybean Oil | 7.0 | 7.0 | 7.0 |
| Cellulose | 5.0 | 5.0 | 5.0 |
| Mineral Mix | 3.5 | 7.0 | 7.0 |
| Vitamin Mix | 1.0 | 2.0 | 2.0 |
| L-cystine | 0.3 | 0.6 | 0.6 |
| Choline Bitartate | 0.25 | 0.5 | 0.5 |
| t-Butylhydroquinone | 0.0014 | 0.0028 | 0.0028 |
| Octacosanal | - | - | 7mg/head/day |

### < Evaluation of breaking energy >

The produced standard feed, and restricted feeds (1) and (2) were given to a rat osteoporosis model (Sun et al., J. Jap. Soc. Nutrition and Food Science, 55 (3), 149-155, 2002), and breaking energy was evaluated by the following method.

Fifteen (15) female rats (available from Japan SLC, Inc., Wister, 6-week old at the start of experiment; weight:100 g to 110 g) were prepared. The rats were housed iundividually as one rat per cage and preliminarily fed for one week under the conditions of temperature 25 ± 2°C, relative humidity 60 ± 5%, and light-dark cycle 12 hours. During this period, rats were fed with the standard feed and tap water *ad libitum,* and the average daily intake per head (g/head/day) was calculated.

The 15 rats were divided into three groups (control group, restricted feed group, and test group) with each group having 5 rats, such that the average weight of each group was almost the same. The control group was fed with the standard feed *ad libitum;* the restricted feed group was fed with the restricted feed (1) with half the amount of the above-mentioned average intake, one time per day at 11:00 in the morning; and the test group was fed with the restricted feed (2) in the same way as the restricted feed (1). Each group was fed with the tap water *ad libitum*.

After a 4-week feeding under the conditions, each rat was anesthetized with sodium pentobarbitone, exsanguinated from the abdominal aorta, and killed. Then, each rat was grossly examined at autopsy, and both femurs were collected. Breaking energy was measured using the collected right femur according to the Peng et al.'s method (J. Bone Miner. Res., 2, 249-257, 1987).

### < Evaluation of bone density >

The collected left femur was measured by dual energy X-ray absorptiometry (bone-density measuring equipment DCS-600, available from Aloka Co., Ltd.) at a pitch of 1.0 mm, the image was manipulated, and the bone density was calculated.

### < Concentration of serum estradiol >

The sampled blood was centrifuged to separate and collect serum, and the concentration of estradiol was measured using an Estradiol EIA Kit (available from Funakoshi Co., Ltd.) according to a common procedure.

**Table 2**

| | Comparative Example 1 | Comparative Example 2 | Example 3 Example 3 |
|---|---|---|---|
| | Standard Feed | Restricted Feed (1) | Restricted Feed (2) |
| Breaking Energy (10⁻³ J) | 27.64 | 18.06 | 26.34 |
| Bone Density of Femur (g/cm²) | 0.107 ± 0.02 | 0.060 ± 0.01 | 0.083 ± 0.017 |
| Concentration of Serum Estradiol (µg/ml) | 2.48 | 1.68 | 1.48 |

As shown in Table 2, in case of the group fed with the restricted feed (1), compared with the group fed with the standard feed, breaking energy was low, indicating easy bone fracture. However, in case of the group fed with the restricted feed (2), it was found that the breaking energy was almost the same as that of the group fed with the standard feed. Further, as for the bone density of femur, similarly to the measurement result of the breaking energy, it was found that the bone density of the group fed with the standard feed was almost the same as that of the group fed with the restricted feed (2).

As for the concentration of serum estradiol, no difference between the group fed with the restricted feed (1) and the group fed with the restricted feed (2) was observed.

### Industrial Applicability

The invention can provide a composition containing a higher fatty acid derivative which can be easily produced at low cost, is effective as an agent for prevention and treatment of osteoporosis induced by various factors such as diet, dietary restriction, etc., is highly safe, and can be used regularly; and foods and drinks which comprise the composition, are effective as agents for prevention and treatment of osteoporosis induced by various factors such as diet, dietary restriction, etc., and are suitable as foods and drinks for hospitals, healthy drinks or foods, feeds, etc.

## Claims

1. A composition containing a higher fatty acid derivative, comprising at least one of higher fatty acid derivatives as an active ingredient,
wherein the composition containing a higher fatty acid derivative is used as an agent for prevention and treatment of osteoporosis.

2. The composition containing a higher fatty acid derivative according to claim 1, wherein the at least one of higher fatty acid derivatives comprises a higher fatty acid derivative having a carbon number of 24 to 38.

3. The composition containing a higher fatty acid derivative according to claims 1, wherein the at least one of higher fatty acid derivatives comprises octacosanal.

4. The composition containing a higher fatty acid derivative according to claim 3, wherein the at least one of higher fatty acid derivatives comprises octacosanol.

5. A food and drink comprising the composition containing a higher fatty acid derivative of claims 1.
